# EUROPEAN PATENT SPECIFICATION

(11) **EP 3 170 474 B1**
(45) Date of publication and mention of the grant of the patent: **01.05.2019**
(21) Application number: 16197820.0
(22) Date of filing: 26.08.2010
(51) Int. Cl.: A61F 2/16

(54) **INTRAOCULAR IMPLANT**
INTRAOKULARES IMPLANTAT
IMPLANT INTRA-OCULAIRE

(43) Date of publication of application: 24.05.2017
(62) Divisional of application: 10251496.5
(73) Proprietor: Stevens, Julian Douglas, London N1 7RH (GB)
(72) Inventor: Stevens, Julian Douglas, London N1 7RH (GB)
(74) Representative: Miller Sturt Kenyon

(56) References cited:
- WO-A1-2007/082342
- US-A- 5 171 320
- US-A- 5 697 973
- US-A1- 2004 215 340
- US-A1- 2006 047 339

## Description

### FIELD OF THE INVENTION

The invention relates to an intraocular implant, and in particular to an intraocular implant for use in cataract operations or refractive crystalline lens extraction operations generally.

### BACKGROUND OF THE INVENTION

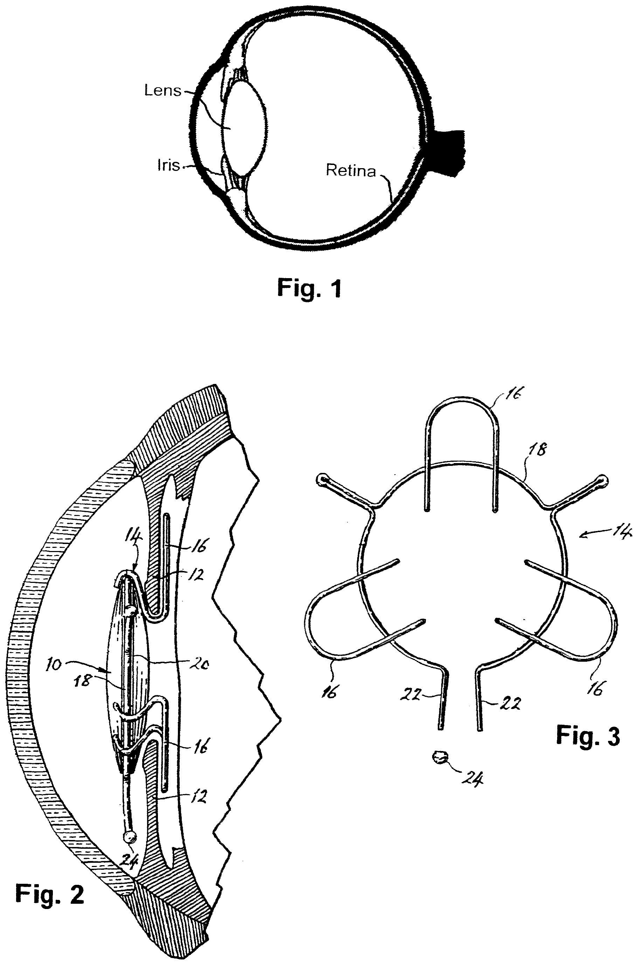

The cataract condition is a well-known eye ailment, which these days is easily treatable through surgery. The condition involves an opacification of the lens (see Fig. 1), which is situated just behind the iris and serves to focus the incoming image onto the retina at the back of the eye.

The surgical procedure involves the removal of the opaque lens and its substitution by an artificial lens having the required focusing effect.

One way of achieving this is described in U.S. patent no. 3,925,825. Figs. 2 and 3 correspond to Figs. 1 and 2 of this patent, and show the placing of a lens 10 anterior to the iris 12. The lens is held in place against the iris by a haptic section 14. The haptic section 14 consists of a series of bent wire loops 16, which are attached to a circular wire frame 18. The lens 10 has a flat circumferential face 20 and the lens is held within the frame 18 by inserting the lens inside the frame 18 and closing the legs 22 of the frame so that the frame 18 closes tightly against the lens face 20. Once the legs 22 have been brought together, their ends are kept together via a terminal part 24.

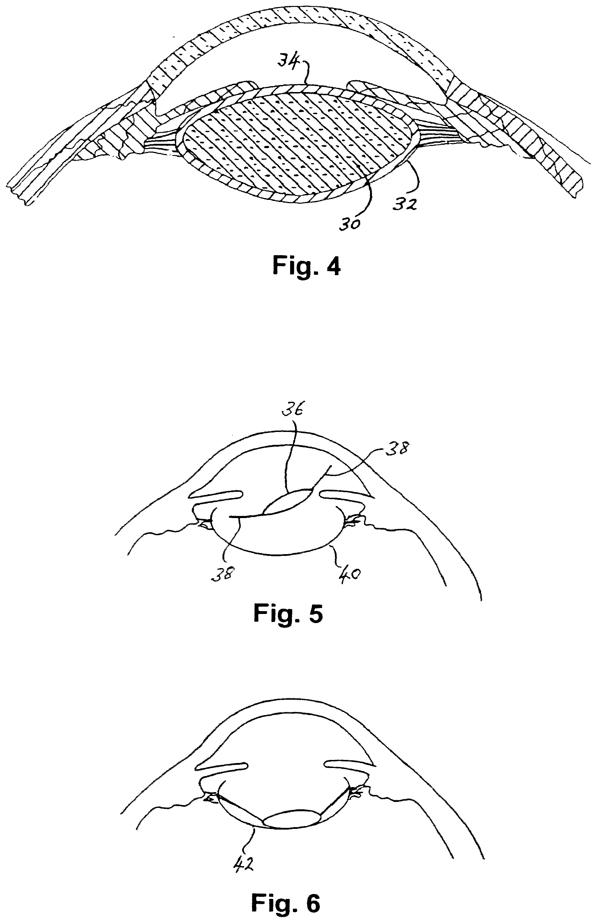

One way of extracting the opaque natural lens - a process known as extra-capsular extraction - will now be described. Reference is first made to US 2003/0130732, Fig. 1 of which corresponds to Fig. 4 of the present application. The lens 30 is accommodated within a so-called capsule, which is shown as item 32 in Fig. 4. To remove the opaque lens, an opening (a so-called "capsulotomy") is made in the anterior part 34 of the capsule 32 manually by a surgeon or by a pulsed laser and the lens 30 is removed through the opening.

To facilitate the removal of the opaque lens, the lens is first emulsified by the phacoemulsification method or by a pulsed laser. Phacoemulsification involves making a small incision in the cornea and introducing a very thin needle through the incision, which is then brought into contact with the lens through the capsulotomy. The needle is caused to vibrate at an ultrasonic frequency by the use of a magnetostrictive driver. The ultrasonic vibrations of the needle soften the lens and emulsify it. The emulsified parts can then be aspirated out of the capsule through the incision. Finally, the incision is widened sufficiently to introduce the substitute artificial lens into the capsule.

A pulsed laser can be used to create an opening in the capsule, by photoablating capsular tissue along a boundary having a predetermined profile, e.g., circular, oval or elliptical.

Like the lens of Fig. 2, the lens in the extra-capsular method is conventionally held within the capsule by the use of haptics. One example of this is shown in US 5,376,115. Figs. 5 and 6 are an extract from this patent, in which the artificial lens 36 with its haptics 38 is introduced through the iris and into the capsule 40 via the capsulotomy (Fig. 5) and finally brought to bear against the inside posterior surface of the capsule (Fig. 6). The haptics 38 are used to centre the lens and secure it in place inside the capsule.

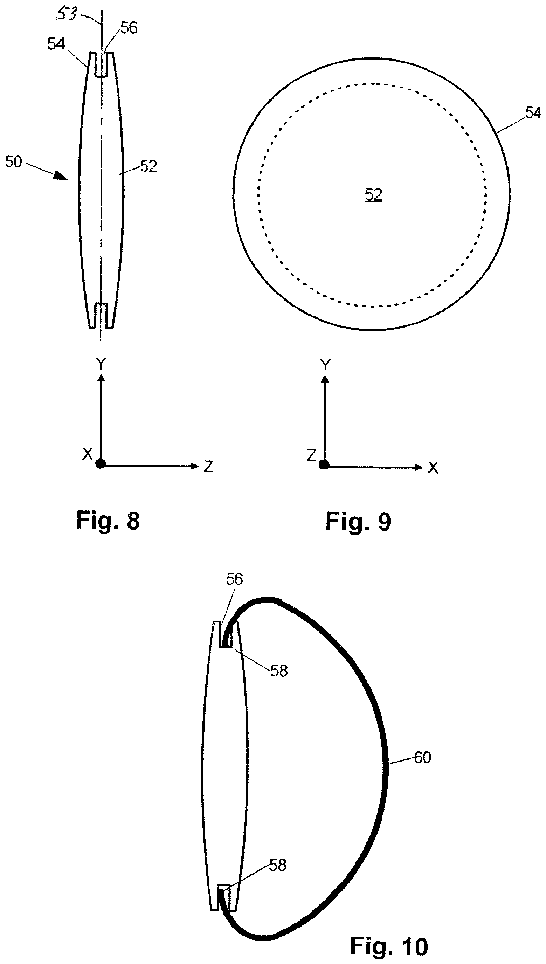

One problem associated with these known capsular insert techniques is that it is difficult to centre the lens accurately in the X-Y plane of the eye. The X-Y-Z co-ordinates are shown in Figs. 8 and 9, where the radius of the lens lies in the X-Y plane and the Z-direction is parallel to the optical axis of the eye. A second problem is that it can be difficult to properly define the position of the lens in the Z-direction. This can lead to difficulties in accurately defining the optical power required for the artificial lens. A third problem, which one embodiment of the present invention can solve, is that, some time after the cataract operation has been carried out, the posterior wall of the capsule (the "posterior capsule") can become opaque like the original lens, requiring a further surgical intervention.

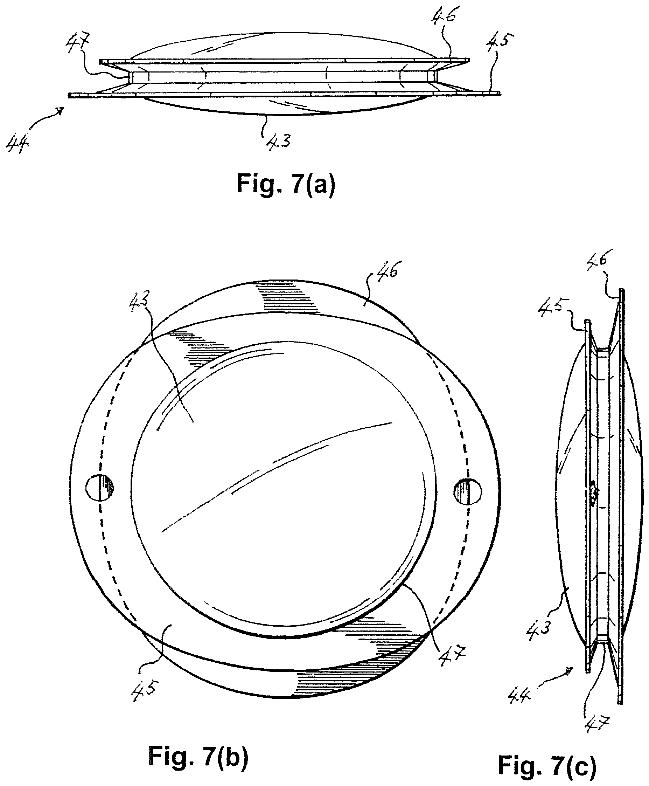

United States Patent no. 6,027,531 describes a so-called "bag-in-lens" technique, in which the optical implant takes the form shown in Figs. 7(a)-7(c). This implant comprises a lens optic 43 and a haptic portion 44 comprising an anterior lip 45 and a posterior lip 46. The haptic portion 44 is configured to form a groove 47, into which will be fitted the lips of a pair of capsulotomies formed respectively in the anterior and posterior walls of the capsule.

US 5,697,973 discloses an intraocular implant for engagement with either the iris or a capsulotomy, the implant consisting of a lens inside a grooved frame portion.

US 5,171,320 shows an intraocular implant comprising an integral lens portion and a peripheral annular groove portion for engagement with a capsulotomy. Other alternative configurations are shown.

WO 2007/082342 discloses an implant that is intended to be inserted into an anterior capsulotomy. The implant comprises a peripheral member with a groove formed by a pair of flanges. The central part of the implant is an opening. An IOL having a series of equidistantly spaced protrusions 51 is inserted into the opening and the protrusions engage with corresponding openings made in the groove.

US 2006/0047339 discloses a device for placement in a capsulorhexis. It includes a flexible ring adapted to fit inside the capsule opening and has a groove for retention of the anterior or posterior capsule in the groove. Eyelets are placed on the ring for suturing the device to an iris or other eye tissue. Cleats can be used to attach a replaceable lens to the ring.

US 2004/0215350 discloses an intraocular lens implant including a lens and haptics extending radially outward from the lens and being formed integrally with the lens. The haptics include an arm and a supporting element for support around the edge of the capsular bag.

### SUMMARY OF THE INVENTION

The invention is defined in claim 1. Further aspects and preferred embodiments are defined in the appended claims. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

The present invention provides an intraocular implant for placement in the eye, the implant comprising an optic constituting a lamina extending along a plane and having a first side and a second side separated by a thickness, wherein first projections extend at discrete locations from a periphery of the implant on the first side and second projections extend at discrete locations from a periphery of the implant on the second side; the first and second projections are formed of the same material as the optic; and a groove lying substantially parallel to the plane is formed in the thickness direction of the lamina between the first and second projections for engagement with the lip of a single capsulotomy only formed in the lens capsule of the eye such that the lip of the lens capsule of the eye is fitted between the first and second projections to secure the implant at a predetermined position in the thickness direction in the eye.

Preferably, the groove extends continuously around the peripheral portion of the implant.

Preferably, the groove is provided at discrete locations around the peripheral portion of the implant.

More preferably, the discrete locations are substantially equidistant from each other.

Preferably, at least one of the first and projections is provided as a plate.

Preferably, the plate is rounded at its end.

Preferably, the implant comprises a multi-lamina unit comprising first and second laminae spaced apart from each other in a direction orthogonal to the planes of the laminae.

Preferably, the implant further comprises at the peripheral portion thereof at least one lug for engagement with corresponding voids formed in the capsule.

Preferably, there are two, three or four lugs spaced around the peripheral portion for engagement with respective voids formed in the capsule.

Preferably, the lugs are substantially equidistantly placed around the peripheral portion of the implant.
Preferably, the implant is a lens.

### BRIEF DESCRIPTION OF THE DRAWINGS

Embodiments of the present invention will now be described, by way of example only, with the aid of the drawings, of which:
Fig. 1 is a sectional view of the human eye;
Fig. 2 is a part-side and part-sectional view of a human eye with an artificial lens substituted for the original lens in accordance with a known method;
Fig. 3 shows a haptic arrangement used in the known method of Fig. 2;
Fig. 4 is a sectional view of a human eye showing the natural lens in its capsule;
Figs. 5 and 6 are sectional views showing two stages in a known lens replacement method employing extra-capsular extraction;
Figs. 7(a)-7(c) are orthogonal views of a known "bag-in-lens" implant;
Figs. 8 and 9 are side and front views, respectively, of a first embodiment of an intraocular insert according to the present invention;
Fig. 10 is the side view according to Fig. 8 with the lens engaged with the capsule;
Figs. 11(a) and 11(b) are a side view of a variant of the first embodiment according to the invention;
Figs. 12(a), 12(b) and 12(c) are side and front views of a further variant of the first embodiment according to the invention;
Fig. 13 is a side view of a yet further variant of the first embodiment according to the invention;
Figs. 14 and 15 are front and plan views, respectively, of a second embodiment of an intraocular insert according to the present invention;
Figs. 16(a) and 16(b) are different realizations of the projections shown in Figs. 14 and 15;
Figs. 17 and 18 are front and plan views, respectively, of a variant of the second embodiment;
Fig. 19 shows two different examples of a third embodiment of an intraocular implant according to the present invention;
Fig. 20 is a plan view of an intraocular implant illustrating the principle of a fourth embodiment of the present invention;
Figs. 21 and 22 are plan and front views, respectively, of one realization of the fourth embodiment, and
Figs. 23 and 24 are plan and front views, respectively, of a second realization of the fourth embodiment.

### DETAILED DESCRIPTION OF THE EMBODIMENTS

A first embodiment of an intraocular implant in accordance with the present invention is illustrated in Figs. 8 and 9, in which Fig. 8 is a side view and Fig. 9 is a front view of the implant.

The implant in this embodiment is an artificial lens implant 50 for treating, e.g., cataracts, and comprises a lens body portion 52, which is the lens proper (the "optic"), and a peripheral portion 54. The peripheral portion 54 has formed in an axially approximately central part thereof (i.e. referred to the Z-axis) a groove 56, which extends in a radial direction from the periphery of the implant toward its optical axis. Thus, the groove 56 lies parallel to, and preferably in, the axially central plane 53 of a lamina constituted by the optic 52. The term "lamina" refers to a plate-like member having a surface area much greater than its thickness, especially at its outer edge. During the surgical procedure, the lens 50 is offered up to the anterior capsulotomy, which has been formed by laser in the capsule 60 (see Fig. 10), as mentioned above in connection with the known implantation method, and the lip 58 of the capsulotomy is fitted into the groove 56, thereby holding the implant in place. Hence, this method dispenses with the need for haptics, instead relying on the lens itself for securing to the capsule. In addition, the correct positioning of the lens vis-à-vis the optical axis of the eye can be ensured by accurately locating the capsulotomy in the X-Y plane (see Figs. 8 and 9). Thus, some eccentricity can be introduced into the radial positioning of the lens by this appropriate locating of the capsulotomy in the capsule.

It is desirable not only that the capsulotomy be accurately located in the capsule, but also that the size of the capsulotomy be accurately defined, so that it can fit the groove in the implant. This is possible at this present time, due to the existence of pulsed ultrafast femtosecond laser technology, by means of which extremely accurate cutting operations can be achieved in a reliable and repeatable manner.

Where desired, one or more fenestrations can be made in the capsule, in order to provide for the passage of a lens or device into the capsular bag or to allow the transfer of aqueous fluid.

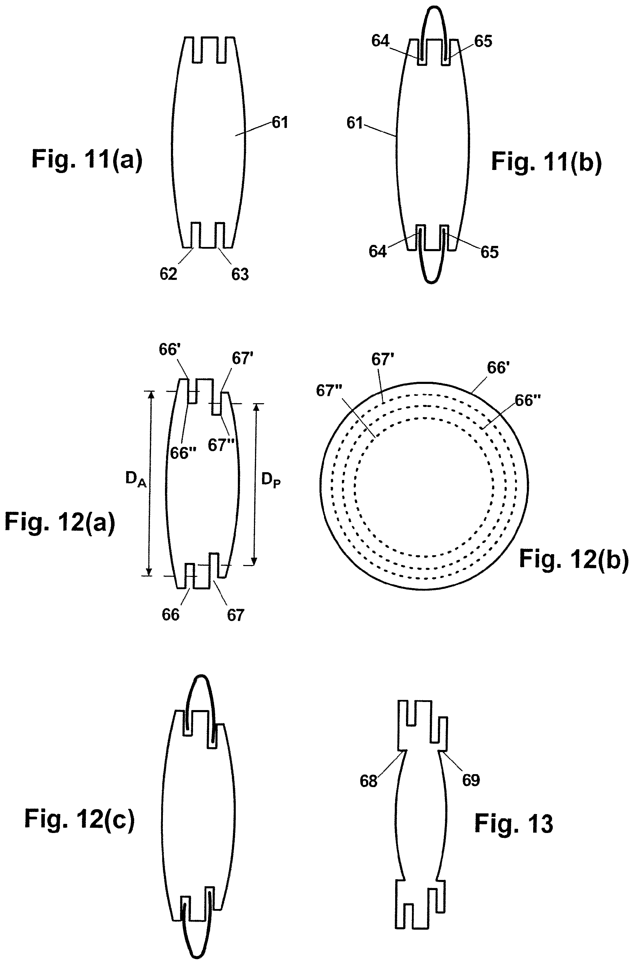

Although this embodiment has been described in connection with an anterior capsulotomy, in practice the implant may be made to engage with a posterior capsulotomy instead, or even with both types of capsulotomy at the same time. A realization of this latter arrangement is illustrated in Figs. 11a and 11b, in which an implant comprises the optic portion 61 and a peripheral portion with a pair of annular grooves 62 and 63. These grooves are spaced apart from each other in the Z-direction (see Fig. 8) and are designed to take respective lips 64, 65 of an anterior and posterior capsulotomy (see Fig. 11b). A variant version of this arrangement is shown in the side and front views of Figs. 12a and 12b, respectively. In this version the posterior capsulotomy is smaller than the anterior capsulotomy. To accommodate this, the anterior groove 66 has a larger mean periphery than the posterior groove 67. This is best explained with reference to Fig. 12b, in which the larger groove has an outer periphery 66' and an inner periphery 66", while the smaller groove has an outer periphery 67' and an inner periphery 67". The mean periphery of the groove 66 is clearly larger than that of the groove 67. For a circular profile, as illustrated, this means that the mean anterior diameter DA of the groove 66 is larger than the mean posterior diameter DP of the groove 67.

This version as fitted to the bag is shown in Fig. 12c.

The Fig. 12 arrangement can be beneficial to a patient, since it is often desirable to limit the extent of the posterior capsulotomy, as this reduces risk of the lens implant being accidentally displaced through the posterior capsulotomy into the vitreous cavity.

One potential drawback of the arrangements illustrated in Figs. 11 and 12 is that the provision of two grooves in the same lens can increase the thickness of the optic. To avoid this, the configuration of Fig. 13 may be employed. In this configuration the peripheral portion of the implant has either or both of an anterior overhang 68 and a posterior overhang 69, which allows a reduced thickness of the optic.

This first embodiment also envisages the use of a complex-lens arrangement involving the use of more than one optic. In particular, two optics may be used, which are connected together to form a single unit. This apparatus may be fixated to the anterior capsulotomy or to a posterior capsulotomy, or to both. In the latter case, the two capsulotomies engage with two different spaced-apart grooves.

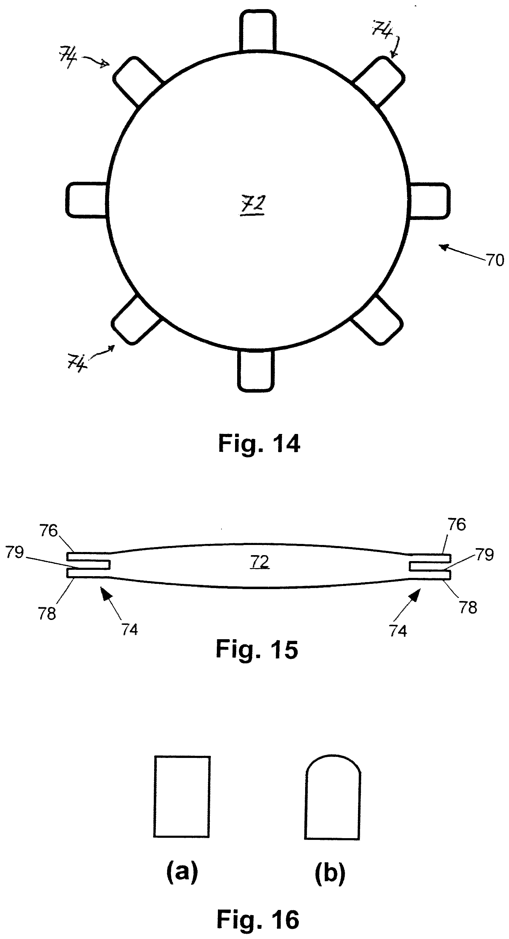

A second embodiment of the invention is illustrated in Figs. 14 and 15. Here a lens 70 has an optic portion 72 and a series of projection portions 74 projecting from the circumference of the lens portion. Referring to the plan view of Fig. 15, each projection portion comprises a first projection 76 and a second projection 78, which are disposed at opposite sides of the lens, and a gap 79 between the first and second projections. The gap 79 corresponds to the groove 54 in Fig. 8. The projections 76 and 78 may be both rectangular or rounded in shape, or a combination of both (see Figs. 16(a) and 16(b)). Hence the projections 76 may be rectangular, while the projections 78 are rounded, or vice-versa, or any combination of the two around the periphery of the implant, or the projections may be all of one type. In practice, the rounded configuration is preferred for all of the projections, since this makes it easier to fit the capsulotomy to the implant and also reduces the risk of trauma to the capsule, that a sharp edge of the rectangular profile might cause. The projections are either integral to the optic (i.e. moulded), or they may be attached to the body of the lens by some suitable means (e.g. mechanical slot and groove) and can be of the same material as the lens optic or of a different bio-material. The projections are either in the form of thin loops or plates. Although eight projection pairs have been shown, the invention is not limited to this number. More or less than eight may be used. The criterion for selecting the appropriate number is the reliability and stability of the attachment of the lens to the capsule.

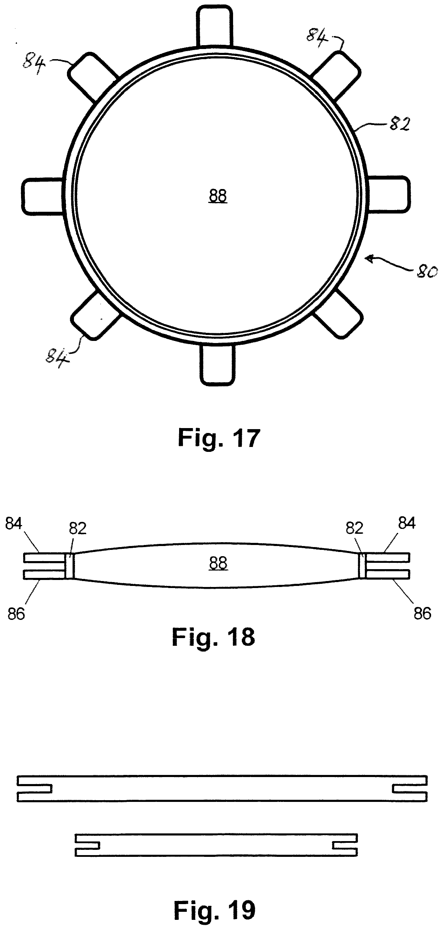

In a variant of the embodiment just described, the projections are not integral with the optic, or attached directly to the optic, but instead form part of a separate projection unit, which is fitted to the optic. This is shown in Figs. 17 and 18, in which the projection unit 80 comprises an annular frame 82, which is tightly fitted to the optic 88, and attached to which are pairs of projections 84, 86.

In a third embodiment of the present invention, the intraocular implant of the first embodiment is not a lens, but a plug or bung. Such an implant is not required to have any optical power, but serves merely to close a fenestration or other aperture in the capsule. An example of this is shown in Fig. 19. Indeed, Fig. 19 shows two examples of different diameter, depending on the size of the fenestration to be closed. It can be seen that this implant is flat and therefore has no optical power. Depending on the position of the fenestration being closed, the plug may also be opaque.

Fenestrations that might be closed off using the plug are, for example, fenestrations introduced into the capsule in order to facilitate the entry of an instrument for evacuating the lens material. In this respect, femtosecond lasers are often used to cut the lens into very small cubes or slices, so that they can be evacuated through a small-bore instrument or cannula. The cannula can be introduced into the capsule through such a fenestration. In addition, the plug can be used to provide tectonic support, in order to keep compartments in the eye physically separate. It is also useful as a means of preventing silicone oil from moving forward into the anterior segment of the eye.

A fourth embodiment will now be described.

The fourth embodiment recognizes that there is often a need to rotationally align a lens implant, where there is an asymmetrical optic (e.g. where there is variable optical power in the lens or where there is a toric optic). This embodiment utilizes forward or backward facing lugs, which are placed in voids (e.g. fenestrations or very small capsulotomy holes) in the capsule. These voids are located radially outside the capsulotomy and can be formed by photoablation via a laser.

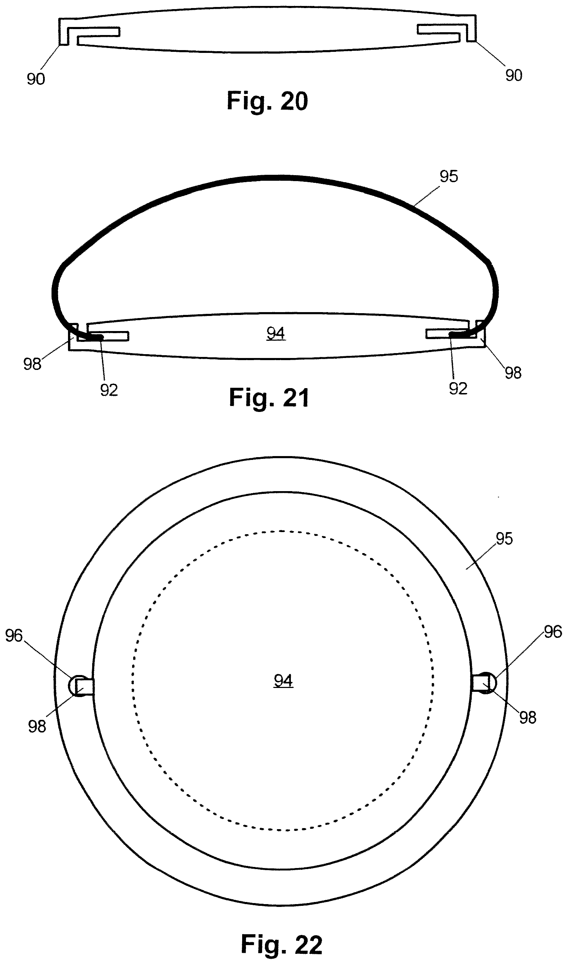

The principle of this embodiment is illustrated in Fig. 20. In Fig. 20 a lens implant such as shown in Fig. 8 or Fig. 15 is provided with one or more lugs 90 situated radially outwardly of the outer ends of the groove 56 or projections 76, 78.

Figs. 21 and 22 illustrate one specific realization of this embodiment, based on the first embodiment. As in Fig. 10, the lip 92 of the capsulotomy in this embodiment is engaged in the groove of the lens 94. Holes 96 have been formed in the capsule 95 and a pair of oppositely disposed lugs 98, which are integral with the lens, are made to engage with these holes, thereby securing the lens in a given rotational position relative to the capsule. In this arrangement the implant is oriented so that the lugs face toward the back of the eye. Instead, the lens may be reversed, so that the lugs face toward the front of the eye and therefore engage with the residual anterior capsule from an inside surface thereof instead of from the outside surface thereof, as in Fig. 21.

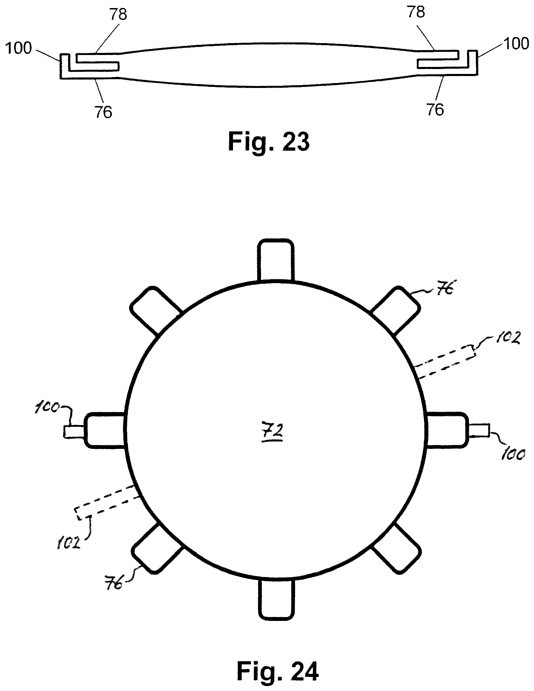

A variant of this arrangement is shown in Figs. 23 and 24, and is based on the second embodiment. Here a pair of oppositely placed lugs 100 is provided as integral extensions to two of the first projections 76 or second projections 78. These engage with holes or voids in the capsule similar to those shown in Fig. 22. In a further variant of this variant arrangement, the lugs are instead each provided between two adjacent projections 76 or 78, as shown by the dotted-line projections 102 in Fig. 24. These projections 102 are therefore dedicated solely to carrying the lugs, whereas in the first arrangement the projections 76 (or 78) act both as protrusions for engagement with the lip of the capsulotomy and as carriers of the lugs for engagement with the capsule.

Although the lugs shown in Figs. 20, 21 and 23 are of a length such as to end up approximately flush with the radially outer part of the projecting portion - e.g. flush with the projections 78 in Fig. 23, and similarly in Fig. 21 - in practice the lugs will have a length such as to enable them to be readily fitted into the voids in the capsule wall, with at the same time the capsule lip being fitted into the groove(s). As shown in Fig. 21, the lugs preferably pass right through through-holes made in the capsule wall, and will therefore be of a length, which enables them to do that, while not being so long that they make it difficult to fit the capsule lip into the groove(s).

As regards the end-profile of the lugs (that is, looking down onto the end of the lugs), this is preferably circular, though other profiles may also be suitable, provided they are able to engage with the voids made in the capsule.

Instead of two lugs per lens, only one may be employed, or more than two - e.g. three or four. They will generally be equally distributed around the periphery of the lens.

The advantages of the present invention are as follows:
- accurate positioning of the lens in the X-Y plane over the pupil centre or any desired centre-point (e.g. the line of sight);
- potentially more accurate placement in the Z-direction, due to the siting of the lens in the anterior or posterior part of the capsule;
- if the lens is supported by the posterior part of the capsule, then the opacification, to which the posterior capsule is often subject post-op, cannot occur;
- the insert can take the form of a plug instead of a lens, which can be used to seal a capsular opening used during surgery;
- such a plug could comprise a valve arrangement to allow the passage of fluid, which could regulate the volume of the capsule;
- because there is predictable positioning of the lens in the Z-direction, then a more reliable biometry formula can be written;
- since the insert is secured by the rim of the capsulotomy, its rotational position can be defined by the forming of marker voids or fenestrations in the capsule and of lugs as extensions to the lens, which engage with the fenestrations;
- the provision of two grooves axially spaced apart in the implant allows anterior and posterior capsulotomies of different sizes to be engaged with the implant, since each capsulotomy is allocated to its own groove.

Although the implant has been described for use with the human eye, it could equally well be used with the animal eye.

In addition, although implants of circular profile have been shown in connection with the various embodiments, the profile used can be any desired profile thought to be appropriate to the situation in question. Thus, oval or elliptical profiles are possible, for instance.

As regards the materials used for the implant any of the materials used for intraocular lens implants, such as acrylic silicone or hydrogel, are suitable.

The implant of the present invention has been described in connection with its use in cataract operations. However, it may also be used for treating myopia, hyperopia, astigmatism or presbyopia (refractive lens exchange surgery).

The foregoing description has been given by way of example only and it will be appreciated by a person skilled in the art that modifications can be made without departing from the scope of the present invention.

## Claims

1. An intraocular implant (70) for placement in the eye, the implant comprising an optic (72) constituting a lamina extending along a plane (53) and having a first side and a second side separated by a thickness, wherein a series of projection portions (74) project from the circumference of the optic, each projection portion comprising a first projection (76) that extends from a periphery of the implant on the first side, and a second projection (78) that extends from a periphery of the implant on the second side; the first and second projections are formed of the same material as the optic; and
a groove (79) lying substantially parallel to the plane is formed in the thickness direction of the lamina between the first and second projections of each projection portion for engagement with the lip of a single capsulotomy only formed in the lens capsule of the eye such that the lip of the lens capsule of the eye is fitted between the first and second projections of each projection portion to secure the implant at a predetermined position in the thickness direction in the eye.

2. An intraocular implant as claimed in claim 1, wherein the projection portions are substantially equidistant from each other.

3. An intraocular implant as claimed in any one of the preceding claims, wherein at least one of the first and second projections is provided as a plate.

4. An intraocular implant as claimed in claim 3, wherein the plate is rounded at its end.

5. An intraocular implant as claimed in any one of the preceding claims, wherein the implant comprises a multi-lamina unit comprising first and second laminae spaced apart from each other in a direction orthogonal to the planes of the laminae.

6. An intraocular implant as claimed in any one of the preceding claims, wherein the implant further comprises at the peripheral portion thereof at least one lug for engagement with corresponding voids formed in the capsule.

7. An intraocular implant as claimed in claim 6, comprising two, three or four lugs spaced around the peripheral portion for engagement with respective voids formed in the capsule.

8. An intraocular implant as claimed in claim 7, wherein the lugs are substantially equidistantly placed around the peripheral portion of the implant.

9. An intraocular implant as claimed in any one of the preceding claims, wherein the implant is a lens.

## Patentansprüche

1. Intraokulares Implantat (70) zur Platzierung in dem Auge, wobei das Implantat eine Optik (72) umfasst, die eine Lamelle bildet, welche sich entlang einer Ebene (53) erstreckt und eine erste Seite und eine zweite Seite aufweist, die durch eine Dicke getrennt sind,
wobei eine Reihe von Vorsprungsabschnitten (74) von dem Umfang der Optik hervorstehen, wobei jeder Vorsprungsabschnitt einen ersten Vorsprung (76), der sich von einem Umfang des Implantats auf der ersten Seite erstreckt, und einen zweiten Vorsprung (78) umfasst, der sich von einem Umfang des Implantats auf der zweiten Seite erstreckt;
wobei der erste und der zweite Vorsprung aus dem gleichen Material wie die Optik gebildet sind; und
eine Nut (79), die im Wesentlichen parallel zu der Ebene liegt, in der Dickenrichtung der Lamelle zwischen dem ersten und zweiten Vorsprung jedes Vorsprungsabschnitts zum Eingriff mit der Lippe einer einzelnen Kapsulotomie ausgebildet ist, die nur in der Linsenkapsel des Auges derart ausgebildet ist, dass die Lippe der Linsenkapsel des Auges zwischen dem ersten und dem zweiten Vorsprung jedes Vorsprungsabschnitts angebracht ist, um das Implantat an einer vorbestimmten Position in der Dickenrichtung in dem Auge zu sichern.

2. Intraokulares Implantat nach Anspruch 1, wobei die Vorsprungsabschnitte im Wesentlichen gleich weit voneinander entfernt sind.

3. Intraokulares Implantat nach einem der vorhergehenden Ansprüche, wobei mindestens einer der ersten und zweiten Vorsprünge als eine Platte vorgesehen ist.

4. Intraokulares Implantat nach Anspruch 3, wobei die Platte an ihrem Ende abgerundet ist.

5. Intraokulares Implantat nach einem der vorhergehenden Ansprüche, wobei das Implantat eine Multilamelleneinheit umfasst, die erste und zweite Lamellen umfasst, die in einer Richtung orthogonal zu den Ebenen der Lamellen voneinander beabstandet sind.

6. Intraokulares Implantat nach einem der vorhergehenden Ansprüche, wobei das Implantat ferner an dem Umfangsabschnitt davon mindestens eine Lasche zum Eingriff mit entsprechenden Hohlräumen, die in der Kapsel ausgebildet sind, aufweist.

7. Intraokulares Implantat nach Anspruch 6, umfassend zwei, drei oder vier Laschen, die um den Umfangsabschnitt herum angeordnet sind, um mit den jeweiligen in der Kapsel ausgebildeten Hohlräumen in Eingriff zu treten.

8. Intraokulares Implantat nach Anspruch 7, wobei die Laschen im Wesentlichen in gleichem Abstand um den peripheren Abschnitt des Implantats herum angeordnet sind.

9. Intraokulares Implantat nach einem der vorhergehenden Ansprüche, wobei das Implantat eine Linse ist.

## Revendications

1. Implant intraoculaire (70) pour le placement dans l'oeil, l'implant comprenant un optique (72) constituant une lame s'étendant le long d'un plan (53) et ayant un premier côté et un second côté séparés par une épaisseur
dans lequel une série de parties en saillie (74) se projettent de la circonférence de l'optique, chaque partie en saillie comprenant une première saillie (76) qui s'étend à partir d'une périphérie de l'implant sur le premier côté, et une seconde saillie (78) qui s'étend à partir d'une périphérie de l'implant sur le second côté ;
les première et seconde saillies sont formées du même matériau que l'optique ; et
une rainure (79) étant sensiblement parallèle au plan est formée dans la direction de l'épaisseur de la lame entre les première et seconde saillies de chaque partie en saillie pour la mise en prise avec la lèvre d'une seule capsulotomie uniquement formée dans la capsule du cristallin de l'oeil de telle sorte que la lèvre de la capsule du cristallin de l'oeil s'ajuste entre les première et seconde saillies de chaque partie en saillie pour fixer l'implant au niveau d'une position prédéterminée dans la direction de l'épaisseur de l'oeil.

2. Implant intraoculaire selon la revendication 1, dans lequel les parties en saillie sont sensiblement équidistantes l'une de l'autre.

3. Implant intraoculaire selon l'une quelconque des revendications précédentes, dans lequel au moins l'une des première et seconde saillies sont prévues sous la forme d'une plaque.

4. Implant intraoculaire selon la revendication 3, dans lequel la plaque est arrondie au niveau de son extrémité.

5. Implant intraoculaire selon l'une quelconque des revendications précédentes, dans lequel l'implant comprend une unité à plusieurs lames comprenant des première et seconde lames espacées l'une de l'autre dans une direction orthogonale aux plans des lames.

6. Implant intraoculaire selon l'une quelconque des revendications précédentes, dans lequel l'implant comprend en outre au niveau de sa partie périphérique au moins une patte pour la prise avec les vides correspondants formés dans la capsule.

7. Implant intraoculaire selon la revendication 6, comprenant deux, trois ou quatre pattes espacées autour de la partie périphérique pour la prise avec les vides respectifs formés dans la capsule.

8. Implant intraoculaire selon la revendication 7, dans lequel les pattes sont placées de manière sensiblement équidistante autour de la partie périphérique de l'implant.

9. Implant intraoculaire selon l'une quelconque des revendications précédentes, dans lequel l'implant est une lentille.
